# EUROPEAN PATENT APPLICATION

(11) **EP 4 765 788 A1**
(43) Date of publication of application: **24.06.2026**
(21) Application number: 25224728.3
(22) Date of filing: 17.12.2025
(51) Int. Cl.: H04M 1/60, H04M 1/72409, H04M 1/72412, H04M 1/72433, A61F 11/14, H04R 1/10

(54) **HEARING PROTECTION COMMUNICATION, COMMUNICATION DEVICE WITH WIRELESS AUDIO FUNCTIONALITY CONTROL, AND RELATED METHODS**

(30) Priority: 19.12.2024 EP 24221442
(71) Applicant: FalCom A/S, 2750 Ballerup (DK)
(72) Inventor: KIRKELUND, Jens, DK-2750 Ballerup (DK); VERNER, Thorbjørn Gade, DK-2750 Ballerup (DK)
(74) Representative: Aera A/S

(57) **Abstract**

A communication device for a hearing protection apparatus comprising a hearing protection device is disclosed. The communication device comprises a processing unit, a memory, a radio interface, and a hearing protection device interface. The communication device also comprises a modular button assembly comprising a wireless interface. The processing unit of the communication device is configured to obtain a first input from the hearing protection device and/or transmit a first output to the hearing protection device via the hearing protection device interface. The radio interface comprises a radio connector that comprises terminals for wired connection to a radio unit. The processing unit is configured to obtain a second input from the radio unit and/or transmit a second output to the radio unit via the radio interface.

## Description

The present disclosure relates to hearing protection communication, devices, systems, and methods related thereto. In particular, a communication device for a hearing protection system, a hearing protection system, an accessory device, and a method operating a communication device is described.

### BACKGROUND

Users of hearing protection systems and devices thereof may be in different situations or missions, e.g. during combat, where normal communication e.g. with team members is challenging or detrimental to the success of the mission.

### SUMMARY

Accordingly, there is a need for communication devices, hearing protection systems, and related methods that mitigate, alleviate, or address the shortcomings existing and provide for improved communication within hearing protection.

A communication device for a hearing protection system comprising a hearing protection device is disclosed. The communication device comprises a processing unit, a memory, a radio interface, and a hearing protection device interface. The processing unit is configured to communicate with the hearing protection device via the hearing protection interface, e.g. the processing unit is configured to obtain a first input from the hearing protection device and/or transmit a first output to the hearing protection device via the hearing protection device interface. The radio interface optionally comprises a radio connector. The radio connector comprises terminals for wired connection to a radio unit. The processing unit is configured to communicate with the radio unit via the radio interface, e.g. the processing unit is configured to obtain a second input from the radio unit and/or transmit a second output to the radio unit via the radio interface. The radio interface may be a digital radio interface. The communication device optionally comprises a wireless interface for communicating with an accessory device. The communication device may be configured to receive one or more commands comprising a first command, e.g. from the accessory device via the wireless interface, associate the first command with a first message, and to output a first signal indicative of the first message via the radio interface.

Also, a method of operating a communication device for a hearing protection system comprising a hearing protection device is disclosed, the communication device comprising a processing unit, a memory, a radio interface comprising a radio connector, a hearing protection device interface, and a wireless interface. The method comprises communicating with the hearing protection device, such as obtaining, via the hearing protection device interface, a first input from the hearing protection device and/or transmitting, via the hearing protection device interface, a first output to the hearing protection device. The method comprises communicating with the radio unit, such as obtaining, via the radio interface, a second input from the radio unit and/or transmitting, via the radio interface, a second output to the radio unit. The method comprises receiving, via the wireless interface, one or more commands comprising a first command from the accessory device; associating the first command with a first message; and outputting a first signal indicative of the first message via the radio interface, such as the radio connector.

A hearing protection system may comprise one or more of a hearing protection device, a communication device, and an accessory device as disclosed herein.

It is an advantage of the present disclosure that audio communication via a radio unit is provided for without at user speaking thereby providing clear communication in situations where silence and/or non-movement, e.g. of the jaw/lips/facial muscles, are required.

### BRIEF DESCRIPTION OF THE DRAWINGS

The above and other features and advantages of the present disclosure will become readily apparent to those skilled in the art by the following detailed description of example embodiments thereof, including those made with reference to the attached drawings, in which:
Fig. 1 illustrating an example user of the hearing protection system/communication device according to this disclosure,
Fig. 2 illustrates an example hearing protection system,
Fig. 3 shows a flow diagram of an example method of operating a communication device according to the disclosure, and
Fig. 4 shows a flow diagram of an example method of operating a communication device according to the disclosure.

### DETAILED DESCRIPTION

Various example embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the disclosure or as a limitation on the scope of the disclosure. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described. Furthermore, definitions, ranges, materials, orientations, and further descriptions of various examples are provided.

A communication device is disclosed. The communication device may, for example, be a component or subsystem of a hearing protection system comprising a hearing protection device, such as a headset, e.g., an over-the-ear type or an in-the-ear type hearing protection device. In one or more examples, the communication device is a standalone unit configured to communicate via interfaces, such as connectors and/or wireless interfaces, with other devices and/or units. The hearing protection system may comprise a hearing protection device. A hearing protection system, such as communication device and/or hearing protection device, may be, or may include, electronics and/or hardware configured to cancel, suppress, reduce, and/or mitigate environmental noise in order to protect the ear and/or associated internal organs that support a sense of hearing for the user of the hearing protection device. In one or more example communication devices, the communication may form part of a hearing protection system comprising the communication device, the hearing protection device, and a radio unit as disclosed herein. The hearing protection device may thus comprise mechanical noise suppression or cancellation, such as earmuffs or earplugs. In one or more examples, the hearing protection system, such as communication device and/or hearing protection device, comprises electronic noise suppression or cancellation, such as active noise cancellation technology, which may generate signals to destructively interfere with sound waves from the user's environment. In one or more examples, the hearing protection system, such as communication device and/or hearing protection device, comprises digital signal processing filters tuned or tunable to suppress, mitigate, or prohibit certain sound waves that come from the user's environment. The hearing protection device may comprise one or more microphones and/one or more receivers/loudspeakers, e.g. for provision of audio communication capabilities, e.g. via wired or wireless connection to the communication device.

The communication device may include a processing unit or processing units. A processing unit or units may be or include general or special purpose semiconductor devices, which may control data or information exchanges within the communication device. A processing unit may be an application specific integrated circuit (ASIC), field programmable gate array (FPGA), computer processing unit (CPU) with one or more cores, a graphics processing unit (GPU), a general purpose GPU (GPGPU), or system on a chip (SoC). In one or more examples, a processing unit is a multi-IC package that includes one or more such devices.

The communication device may include a memory or memories of various types. The memory may be fixed and/or removable. Memory may be static or dynamic random access memory (SRAM or DRAM, respectively), NAND, and/or other volatile or non-volatile memory types. In one or more examples, a memory and processor unit are portions of a common semi-conductor device, such as (i) a die or multiple dies with memory and processing capability, (ii) a package on package (POP) assembly, and/or (iii) a multi-chip package (MCP). The processing unit may be configured to read from and/or write to memory.

The communication device may include a radio interface. The radio interface may be seen as an intermediary, which may be hardware or software, or a combination of both, that couples or provides a communication path between a processing unit or processing units and a radio unit or radio units. The radio interface may translate data or control signaling from one protocol to another. The radio interface may be or may include a codec. The radio interface may be a digital radio interface. In one or more examples, the radio interface comprises or is a radio connector, which may include terminals for wired connection to the radio unit or to multiple radio units. In other words, the radio connector may be configured to mechanically and/or electrically connect, such as releasably connect, the communication device to a radio unit.

The radio unit may be seen as one or more wireless chipsets and/or one or more RF chains, which may include processing resources and/or mechanical, electronic, software, and/or firmware to support wireless communication with the wireless communication device. In one or more examples, the radio interface may be referred to as a first interface or simply an interface.

The communication device may include a hearing protection device interface. The hearing protection device interface may be seen as an intermediary, which may be hardware or software, or a combination of both, that couples or provides a communication path between a processing unit and a hearing protection device. The hearing protection device interface may translate data or control signaling from one protocol to another. The hearing protection device interface may be or may include a codec, which may be the same as or different from another codec described herein. In one or more examples, the hearing protection device interface may be referred to as a second interface or simply an interface. The hearing protection device interface may comprise or be a hearing protection device connector. The hearing protection device connector may be configured to mechanically and/or electrically connect, such as releasably connect, the communication device to a hearing protection device. The hearing protection device interface may be a wireless interface.

The communication device may comprise a user interface, e.g. comprising one or more buttons, such as one or more push buttons and/or one or more switch buttons.

The processing unit of the communication device may be configured to manage and/or control operations of or among the various components or subsystems of the communication device. By way of example, the processing unit may be configured to communicate with, such as obtain input from and/or transmit output to, the hearing protection device, e.g. via the hearing protection interface/connector. Thus, the processing unit may be configured to obtain, e.g., receive, a first input from the hearing protection device, e.g. via the hearing protection device interface/connector, and/or transmit a first output to the hearing protection device, e.g. via the hearing protection device interface/connector.

The first input and/or the first output may comprise an audio signal and/or one or more control signals. In other words, the first input may be or comprise an audio input and/or the first output may comprise or be an audio output. The first input may be from one or more microphones of the hearing protection device. The first output may be for one or more loudspeakers of the hearing protection device.

Thus, this exchange and/or communication of first input and/or first output may be via the hearing protection device interface, such as the hearing protection connector, of the communication device.

The processing unit may be configured to communicate with, such as obtain input from and/or transmit output to, the radio unit, e.g. via the radio interface/connector. Thus, the processing unit may be configured to obtain, e.g., receive, a second input from the radio unit, e.g. via the radio interface/connector, and/or transmit a second output to the radio unit, e.g. via the radio interface/connector. The second input and/or the second output may comprise an audio signal and/or one or more control signals. In other words, the second input may be or comprise an audio input and/or the second output may comprise or be an audio output.

In other words, the processing unit may be configured to obtain a second input from the radio unit, e.g. via the radio interface, and/or transmit a second output to the radio unit, e.g. via the radio interface.

In one or more examples, the first output may be based on the second input. In other words, the second input, such as an audio signal, to the communication device from the radio unit may form or be used as basis for the first output, such as an audio signal, to the hearing protection device. In one or more examples, the communication device may be configured to perform one or more of filtering, noise cancellation, and beamforming on the second input from the radio interface for provision of the first output to the hearing protection interface. In one or more examples, the first output may be based on the first input. In other words, first second input, such as an audio signal, to the communication device from the hearing protection device may form or be used as basis for the first output, such as an audio signal, to the hearing protection device. In one or more examples, the communication device may be configured to perform one or more of filtering, noise cancellation, hear-through processing, and beamforming on the first input from the hearing protection device interface for provision of the first output based on the first input to the hearing protection interface. Thus, in one or more examples, the first output is based on the first input and the second input.

In one or more examples, the second output may be based on the first input. In other words, the first input, such as an audio signal, to the communication device from the hearing protection device may form or be used as basis for the second output, such as an audio signal, to the radio unit. In one or more examples, the communication device may be configured to perform one or more of filtering, noise cancellation, and beamforming on the first input from the hearing protection interface for provision of the second output to the radio interface.

In one or more examples, the communication device comprises a wireless interface, e.g. for communicating with one or more of an accessory device, the hearing protection device, and the radio unit. The communication device may be configured to receive one or more commands comprising a first command also denoted Com_1 from the accessory device via the wireless interface. In one or more examples, the communication device may be configured to receive one or more commands, such as the first command, via the user interface, e.g. by a user pressing a button of the user interface. The communication device may be configured to, e.g. in response to receiving the first command, associate the first command with a first message, and to output a first signal indicative of the first message via the radio interface/radio connector. In other words, the communication device may be configured to, e.g. in response to receiving the first command, map the first command to the first message, e.g. select or generate a first message based on the first command. The first message may be an audio message. The first signal may be an audio signal or comprise a message identifier indicative of an audio signal. In other words, the second output may comprise the first signal. A message identifier allows the receiving device or system to retrieve a corresponding message and output the message content. Further, use of a message identifier reduces the required bandwidth in the communication. It may be appreciated that the present disclosure allows for audio communication of the user of the communication device, such as the user of the hearing device system, via a radio unit by providing a mechanical command which is then translated or transformed (such as mapped to) into an audio signal or audio communication without the user speaking thereby providing clear communication in situations where silence and/or non-movement, e.g. of the jaw/lips/facial muscles, are required. For example, the user may respond to an audio communication received from a remote colleague and outputted at the hearing protection device by mechanically activating a user interface of the accessory device which in turn provides a message, such as an audio message to the remote colleague. Thereby, the user may be able to listen to audio messages via the hearing protection device and respond to the audio message without speaking.

The communication device may be configured to receive a second command also denoted Com_2, e.g. from the accessory device via the wireless interface. In one or more examples, the communication device may be configured to receive one or more commands, such as the second command, via the user interface, e.g. by a user pressing a button of the user interface.

The communication device may be configured to, e.g. in response to receiving the second command Com_2, associate or map the second command Com_2 with a second message M_2, and to output a second signal indicative of the second message via the radio interface/radio connector. In other words, the communication device may be configured to, e.g. in response to receiving the second command, to map the second command to the second message, e.g. select or generate a second message based on the second command. The second message may be an audio message. The second signal may be an audio signal or comprise a message identifier indicative of an audio signal. In other words, the second output may comprise the second signal.

In one or more examples, a communication device for a hearing protection system comprising a hearing protection device is disclosed, the communication device comprising a processing unit; a memory; a radio interface; and a hearing protection device interface, wherein the processing unit is configured to obtain a first input from the hearing protection device and/or transmit a first output to the hearing protection device via the hearing protection device interface, and wherein the radio interface comprises a radio connector, where the radio connector comprises terminals for wired connection to a radio unit, the processing unit is configured to obtain a second input from the radio unit and/or transmit a second output to the radio unit via the radio interface, wherein the communication device comprises a wireless interface for communicating with an accessory device, wherein the communication device is configured to receive one or more commands comprising a first command from the accessory device via the wireless interface, to associate the first command with a first message, and to output a first signal indicative of the first message via the radio interface.

The accessory device may comprise a processing unit; a wireless interface; and a user interface, such as one or more buttons, wherein the processing unit is configured to detect a user input via the user interface, and transmit or output a first command to the communication device via the wireless interface in accordance with a detection of a first user input, such as activation of a first button of the user interface, indicative of the first command. In one or more examples, the processing unit of the accessory device is configured to detect a user input via the user interface, and transmit or output a second command to the communication device via the wireless interface in accordance with a detection of a second user input, such as activation of a second button of the user interface, indicative of the second command.

The communication device may comprise a housing. The housing may be referred to, in one or more examples, as a/an package, enclosure, casing, shell, chassis, compartment, container, cabinet, cover, encasement, envelope, body, frame, structure, receptacle, case, assembly, shroud, encapsulation, mounting, or the like. The housing may be referred to, in one or more examples, as a means for housing or a housing means. The housing may comprise one or more recesses, such as a first recess and/or a second recess. The recess may be referred to, in one or more examples, as a/an opening, cavity, depression, indentation, hollow, concavity, void, groove, channel, alcove, aperture, notch, socket, well, pocket, cutout, depression, inset, niche, or the like. The (first) recess may be formed in the outer surface of the housing, e.g. in a first side surface of the housing.

**In** one or more examples, the wireless interface of the communication device is configured to use a Bluetooth protocol, such as a Bluetooth Low Energy (BLE) protocol. In one or more examples, the wireless interface of the communication device comprises a wireless processing unit also denoted a transceiver and an antenna. The wireless processing unit/transceiver may be in a common package with the antenna or portion of the antenna. The wireless processing unit may be connected to an antenna that is located elsewhere. The wireless processing unit may be or may include a wireless chipset. A wireless chipset may be seen as an integrated circuit assembly (such as a semiconductor or package of semiconductors) for wireless communication. The wireless chipset may be an SoC. The wireless chipset may include a modem, controller, and/or RF chain. The wireless chipset may be referred to as an RFIC. The wireless processing unit, for example, in combination with the antenna, may be capable of transmitting and/or receiving wireless data and/or control signals, such as commands. The wireless processing unit may be configured for signal processing. The wireless processing unit may be configured for one or more specific protocols, such as IEEE 802.11 (Wifi), Bluetooth, IEEE 802.15.4 (Zigbee), Digital Enhanced Cordless Telecommunications (DECT), and/or a cellular protocol, such as one operating according to a 3GPP standard.

In one or more examples, the first command comprises a first command identifier, and wherein to associate the first command with a first message optionally comprises to associate the first command identifier with the first message.

In one or more examples, the first message is a pre-determined, such as pre-programmed and/or pre-recorded, message associated with the first command. For example, the first message may be a ready message and the first signal may be an audio signal comprising the audio message "Ready to execute".

In one or more examples, the first message may be a pre-determined voice prompt or a pre-determined text prompt.

In one or more examples, the communication device is configured to receive, via the radio interface, a second radio signal in response to the first signal, and to transmit the first output comprises to transmit the second radio signal via the the hearing protection interface. The second radio signal may be indicative of a confirmation or an abortion associated with the first message.

In one or more examples, to output the first signal indicative of the first message via the radio interface comprises to output the first signal via the radio interface to a radio unit of another user and/or to a server device.

In one or more examples, the communication device is configured to output the first message via the hearing protection interface before outputting the first signal via the radio interface.

The communication device/accessory device may implement a message scrolling capability. Thus, the communication may be configured to receive a third command also denoted a scrolling command from the accessory device via the wireless interface, select and output, via the hearing protection interface and in response to the third command, a next message, e.g. in a circular list or buffer of messages; receive the first command, associate the first command with the next message as a first message, and to output a first signal indicative of the first message via the radio interface. Receipt of further third command(s) without receipt of the first command leads to selection and output of next message(s) in the circular list, thereby allowing a user to scroll through a number of available messages and select which one is to be used as the first signal to be output via the radio interface.

In one or more examples, the communication device is configured to receive a second command from the accessory device via the wireless interface, to associate the second command with a second message, and to output a second signal indicative of the second message via the radio interface. For example, the second message may be an abort message and the second signal may be an audio signal comprising the audio message "Abort task".

In one or more examples, the communication device is configured to receive a second command from the accessory device, e.g. where the second command is configured to confirm or cancel the first command. The communication device may be configured to output the first signal via the radio interface in accordance with the second command being indicative of a confirmation of the first message. In other words, to output the first signal via the radio interface may be dependent or conditioned on receipt of a second command indicative of a confirmation of the first message. The communication device may be configured to forgo to output the first signal via the radio interface in accordance with the second command being indicative of a cancellation of the first message. In other words, to output the first signal via the radio interface may be omitted upon receipt of a second command indicative of a cancellation of the first message. One or both of cancellation and/or confirmation by a second command may be implemented, e.g. with a time out for cancellation or confirmation. Thereby a more failsafe communication is provided.

In one or more examples, an accessory device for a hearing protection system comprising a hearing protection device and/or a communication device is disclosed, the accessory device comprising a processing unit, a wireless interface, and a user interface, wherein the processing unit is configured to detect a user input via the user interface, and transmit or output a first command to the communication device via the wireless interface in accordance with a detection of a first user input, wherein the first command is indicative of a first message.

In one or more examples, the user interface of the accessory device comprises one or more of a press button, a switch button, and a joystick.

In one or more examples, the user interface of the accessory device includes one or more buttons, each of which may be associated with one or more functions or commands. For example, a first button may be linked to or associated with transmission of the first command and/or a second button may be linked to or associated with transmission of the second command. Button, as used herein, may refer to a device operable by a user to activate, deactivate, modulate, change, and/or control a a command, function, aspect, response, and/or operation of the communication device and/or a subsystem or component of the communication device and/or one or more units or devices connected to the communication device. A button, for example, may be a physical input element, such as a/an actuator, input device, control element, manipulable element, interface, user interface, tactile device, manual control, and/or depressible member. A button may have, in one or more examples, a functional description or characteristic, such as user-actuatable, pressure-sensitive, pressure-actuated, touch-responsive, touch-sensitive, manually operated, force-receiving, and/or haptic. A button, for example, may have one or more operational characteristics, such as momentary contact, push-button, depression-activated, toggle/togglable, spring-biased, reciprocating, three-way, contact-making. A button may be a portion of a touch-screen user interface.

In one or more examples, the accessory device is configured to be mounted on a weapon of a user of the communication device. Thereby a user involved in combat or other situations where communication cannot be performed via speaking and/or moving the jaw, lips or facial muscles can provide clear and concise communication via the radio unit/interface. The accessory device may be configured to be mounted on a helmet, glove, or a uniform sleeve of a user of the communication device, e.g. by a mechanical connection or a hook and loop attachment.

Also, a method of operating a communication device for a hearing protection system comprising a hearing protection device is disclosed, the communication device comprising a processing unit, a memory, a radio interface comprising a radio connector, a hearing protection device interface, and a wireless interface. The method comprises obtaining, via the hearing protection device interface, a first input from the hearing protection device; transmitting, via the hearing protection device interface, a first output to the hearing protection device; obtaining, via the radio interface, a second input from the radio unit; transmitting, via the radio interface, a second output to the radio unit; receiving, via the wireless interface, one or more commands comprising a first command from the accessory device; associating the first command with a first message; and outputting a first signal indicative of the first message via the radio interface.

It is noted that descriptions and features of communication device functionality, such as communication device/processing unit configured to, also apply to methods and vice versa. For example, a description of a communication device/processing unit configured to receive also applies to a method, e.g. of operating a communication device, wherein the method comprises receiving and vice versa.

The following figures are schematic and simplified for clarity, and they merely show details which aid understanding the disclosure, while other details have been left out. Throughout, the same reference numerals are used for identical or corresponding parts. The skilled person will recognize that the various definitions, functions, materials, components, and the like described above may be applied to and/or combined with the features described with reference to the figures and, for the sake of brevity, may not be repeated in full.

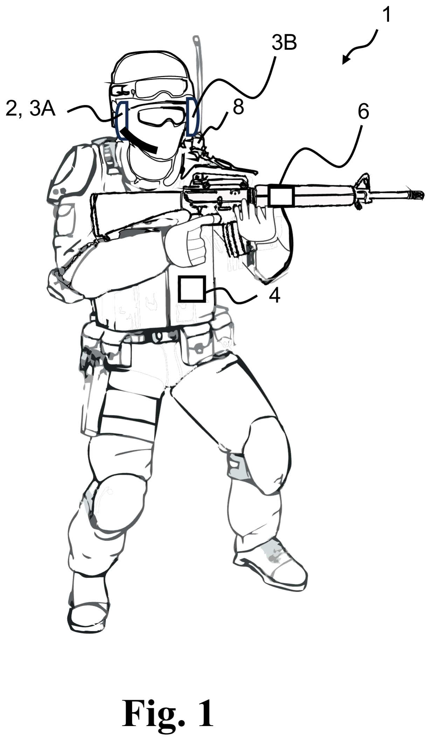

Fig. 1 illustrates for context an example user of the hearing protection system/communication device according to this disclosure. The user wears a hearing protection system 1 comprising a hearing protection device 2, e.g. including first earcup 3A and second earcup 3B; a communication device 4, and accessory device 6. **In** one or more examples, the hearing protection device 2 may be an earplug-type hearing protection device. The communication device 4 is connected to a radio unit 8 with a wire via radio interface/connector of the communication unit 4. Further, the communication device 4 is wirelessly connected to the accessory device 6 via wireless interface of the communication unit 4. The accessory device 6 is optionally mounted to a weapon of the user. Thereby, the user may be able to operate the accessory device 6 without losing control of the weapon.

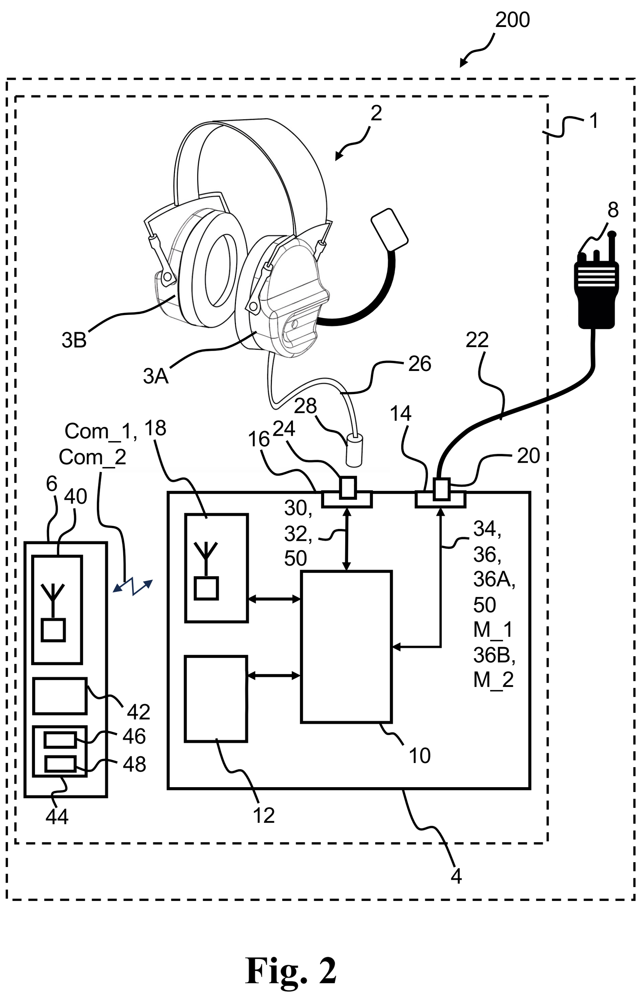

Fig. 2 illustrates an example hearing protection system 1 comprising hearing protection device 2, communication device 4, and accessory device 6, the communication device 4 comprising a processing unit 10; a memory 12; a radio interface 14; a hearing protection device interface 16, and a wireless interface 18. The radio interface 14 comprises a radio connector 20 configured to mechanically and electrically connect the communication device 4 to the radio unit via wire or cable 22 provided with communication device connector (not shown). The hearing protection device interface 16 comprises a hearing protection device connector 24 configured to mechanically and electrically connect the communication device 4 to the hearing protection device 2 via wire or cable 26 provided with communication device connector 28. An alternative hearing protection system 200 is depicted in Fig. 2, which may include the communication device 4, the hearing protection device 2, the accessory device 6, and the radio unit 8.

The processing unit 10 is configured to obtain a first input 30 from the hearing protection device and/or transmit a first output 32 to the hearing protection device via the hearing protection device interface 16/connector 24.

The radio connector 20 comprises terminals for wired connection to the radio unit 8, and the processing unit 10 is configured to obtain a second input 34 from the radio unit 8 and transmit a second output 36 to the radio unit 8 via the radio interface 14/connector 20.

The wireless interface 18 is optionally a BLE interface and is configured to communicate with accessory device 6 via wireless interface 40 of the accessory device 6, wherein the communication device 4/processing unit 10 is configured to receive one or more commands comprising a first command Com_1 from the accessory device 6 via the wireless interface 18.

The communication device 4/processing unit 10 is configured to associate the first command Com_1 with a first message M_1, and to output a first signal 36A indicative of the first message M_1 via the radio interface 14/connector 20 to the radio unit 8. For example, the first message M_1 may be a ready message and the first signal 36A may be an audio signal comprising the audio message "Ready to execute" indicative of the first message. The audio message used as the first signal may be stored in the memory 12 and pre-determined, such as pre-recorded, e.g. by the user prior to the mission.

The communication device 4 may be configured to receive a second command Com_2 from the accessory device 6 via the wireless interface, to associate the second command with a second message M_2, and to output a second signal 36B indicative of the second message via the radio interface 14/connector 20 to the radio unit 8. For example, the second message M_2 may be an abort message and the second signal 36B may be an audio signal comprising the audio message "Abort task".

The accessory device 6 comprises a processing unit 42 and a user interface 44 comprising a first button 46 and optionally a second button 48. The processing unit 42 is configured to detect a user input via the user interface 44, and transmit or output the first command Com_1 to the communication device 4 via the wireless interface 40 in accordance with a detection of a first user input, e.g. activation of the first button 46. The first command Com_1 is indicative of a first message. Optionally, the processing unit 42 is configured to transmit or output a second command Com_2 to the communication device 4 via the wireless interface 40 in accordance with a detection of a second user input, e.g. activation of the second button 48. The accessory device may be configured to be mounted on a weapon of a user of the communication device as shown in Fig. 1.

The first command Com_1 may comprise a first command identifier and to associate the first command with a first message may comprise to associate the first command identifier with the first message. In other words, the processing unit 10 may be configured to map the first command identifier to the first message e.g. by mapping the first command identifier to a location of an audio message to be used as the first signal. Thus, the processing unit 10 may be configured to retrieve an audio message based on the first command/first message. The, first message may be a pre-determined voice prompt or a pre-determined text prompt. The same may apply to the second command Com_2.

The communication device is optionally configured to receive, via the radio interface 14, a second radio signal 50 e.g. in response to the first signal 36A, and to transmit the first output 32 comprises to transmit the second radio signal 50 via the hearing protection interface 16. In one or more examples, the second radio signal is an execution order.

To output the first signal 36A indicative of the first message via the radio interface 14 comprises to output the first signal 36A via the radio interface 14 to a radio unit of another user and/or to a server device.

The communication device 4 may be configured to output the first message via the hearing protection interface before outputting the first signal via the radio interface.

The communication device is configured to receive a second command from the accessory device, where the second command is configured to confirm or cancel the first command.

The operations of the communication device 4 may be considered a method that the communication device 4 is configured to carry out. Also, while the described functions and operations may be implemented in software, such functionality may also (or alternatively) be carried out via dedicated hardware or firmware, or some combination of hardware, firmware and/or software.

Memory 12 may be one or more of a buffer, a flash memory, a hard drive, a removable media, a volatile memory, a non-volatile memory, a random access memory (RAM), or other suitable device, such as described elsewhere herein. In a typical arrangement, memory 12 may include a non-volatile memory for long term data storage and a volatile memory that functions as system memory for processing unit 10. Memory 12 may exchange data with processing unit 10 over a data bus. Control lines and an address bus between memory 12 and processing unit 10, as well as other components of communication device 4, also may be present (not shown in Fig. 1). Memory 12 is considered a non-transitory computer readable medium.

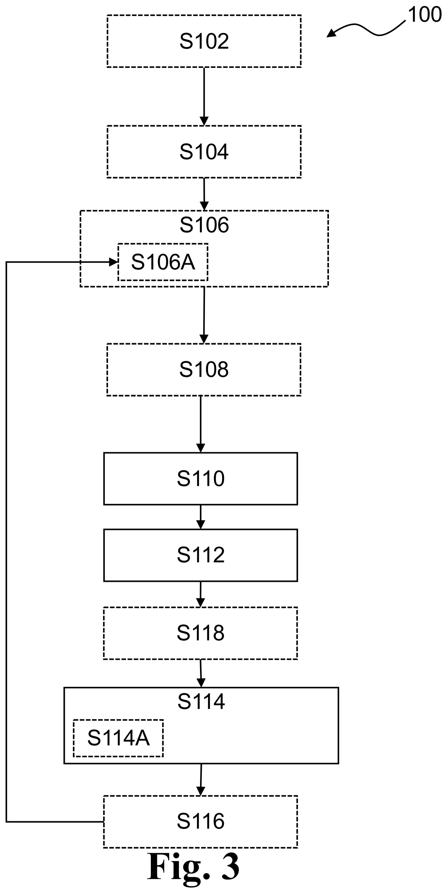

Fig. 3 shows a flow diagram of an example method of operating a communication device according to the disclosure. The method 100 is a method of operating a communication device for a hearing protection system comprising a hearing protection device, the communication device comprising a processing unit, a memory, a radio interface comprising a radio connector, a hearing protection device interface, and a wireless interface. The method 100 comprises obtaining S102, via the hearing protection device interface, a first input from the hearing protection device; transmitting S104, via the hearing protection device interface, a first output to the hearing protection device; obtaining S106, via the radio interface, a second input from the radio unit; transmitting S108, via the radio interface, a second output to the radio unit; receiving S110, via the wireless interface, one or more commands comprising a first command from the accessory device; associating, such as mapping, S112 the first command with a first message; and outputting S114 a first signal indicative of the first message via the radio interface.

The method 100 optionally comprises receiving S116, via the radio interface, a second radio signal in response to the first signal, and wherein transmitting S106 the first output comprises transmitting S106A the second radio signal via the hearing protection interface.

In the method 100, outputting S114 the first signal indicative of the first message via the radio interface may comprise outputting S114A the first signal via the radio interface to a radio unit of another user and/or to a server device.

In the method 100, the method optionally comprises outputting S118 the first message via the hearing protection interface before outputting S114 the first signal via the radio interface.

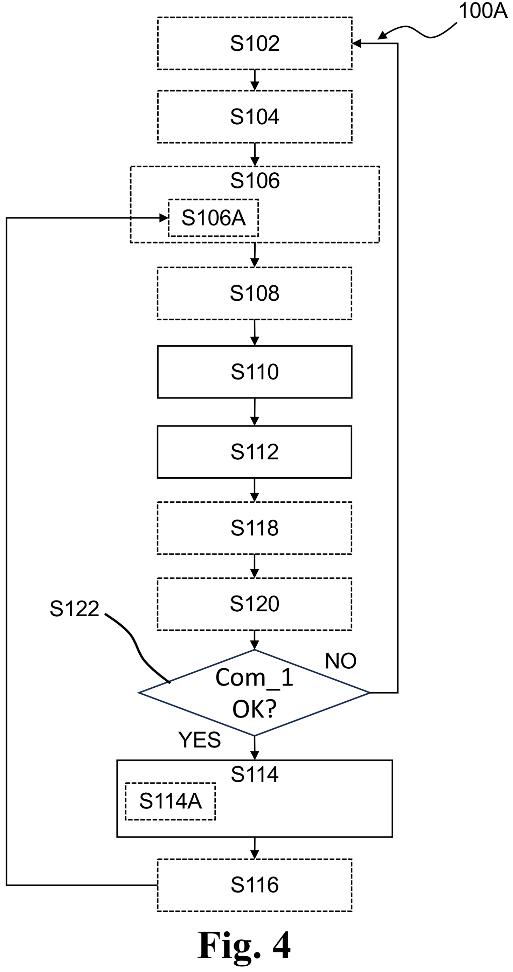

Fig. 4 shows a flow diagram of an example method of operating a communication device according to the disclosure. The method 100A is similar to method 100 and descriptions of features with same reference are not repeated for brevity. The method 100A comprises receiving S120 a second command, e.g. from the accessory device, where the second command is configured to confirm or cancel the first command. The method 100A optionally proceeds to outputting S114A the first signal indicative of the first message via the radio interface in accordance with determining S122 that the first command Com_1 is ok, e.g. if the second command is indicative of a confirmation of the first command or the second command (cancellation) is not received within a time limit. The method 100A optionally forgoes to outputting S114A the first signal indicative of the first message via the radio interface in accordance with determining S122 that the first command Com_1 is not ok, e.g. if the second command is indicative of a cancellation of the first command or the second command (confirmation) is not received within a time limit.

Example communication devices, accessory devices, hearing protection systems, and methods are outlined in the following items.

Item 1. A communication device (4) for a hearing protection system (1) comprising a hearing protection device (2), the communication device (4) comprising:
a processing unit (10);
a memory (12);
a radio interface (14); and
a hearing protection device interface (16),
wherein the processing unit is configured to obtain a first input from the hearing protection device and/or transmit a first output to the hearing protection device via the hearing protection device interface, and wherein the radio interface comprises a radio connector, where the radio connector comprises terminals for wired connection to a radio unit, the processing unit is configured to obtain a second input from the radio unit and/or transmit a second output to the radio unit via the radio interface, wherein the communication device comprises a wireless interface (18) for communicating with an accessory device, wherein the communication device is configured to receive one or more commands comprising a first command from the accessory device via the wireless interface, to associate the first command with a first message, and to output a first signal indicative of the first message via the radio interface.

Item 2. Communication device according to Item 1, wherein the wireless interface is configured to use a Bluetooth protocol, such as a BLE protocol.

Item 3. Communication device according to any one of Items 1-2, wherein the first command comprises a first command identifier, and wherein to associate the first command with a first message comprises to associate the first command identifier with the first message.

Item 4. Communication device according to any of Items 1-3, wherein the first message is a pre-determined message associated with the first command.

Item 5. Communication device according to Item 4, wherein the first message is a pre-determined voice prompt or a pre-determined text prompt.

Item 6. Communication device according to any of Items 1-5, wherein the communication device is configured to receive, via the radio interface, a second radio signal in response to the first signal, and to transmit the first output comprises to transmit the second radio signal via the hearing protection interface.

Item 7. Communication device according to any of Items 1-6, wherein to output the first signal indicative of the first message via the radio interface comprises to output the first signal via the radio interface to a radio unit of another user and/or to a server device.

Item 8. Communication device according to any of Items 1-7, wherein the communication device is configured to output the first message via the hearing protection interface before outputting the first signal via the radio interface.

Item 9. Communication device according to any of Items 1-8, wherein the communication device is configured to receive a second command from the accessory device, where the second command is configured to confirm or cancel the first command.

Item 10. An accessory device for a hearing protection system comprising a hearing protection device and a communication device, the accessory device comprising a processing unit, a wireless interface, and a user interface, wherein the processing unit is configured to detect a user input via the user interface, and transmit or output a first command to the communication device via the wireless interface in accordance with a detection of a first user input, wherein the first command is indicative of a first message.

Item 11. Accessory device according to Item 10, wherein the user interface comprises one or more of a press button, a switch button, and a joystick.

Item 12. Accessory device according to Item 11, wherein the accessory device is configured to be mounted on a weapon of a user of the communication device.

Item 13. A hearing protection system comprising a communication device according to any of Items 1-9 and an accessory device according to any of Items 10-12.

Item 14. A method of operating a communication device for a hearing protection system comprising a hearing protection device, the communication device comprising a processing unit, a memory, a radio interface comprising a radio connector, a hearing protection device interface, and a wireless interface, the method comprising:
obtaining, via the hearing protection device interface, a first input from the hearing protection device;
transmitting, via the hearing protection device interface, a first output to the hearing protection device;
obtaining, via the radio interface, a second input from the radio unit;
transmitting, via the radio interface, a second output to the radio unit;
receiving, via the wireless interface, one or more commands comprising a first command from the accessory device;
associating the first command with a first message; and
outputting a first signal indicative of the first message via the radio interface.

Item 15. Method according to Item 14, wherein the wireless interface is configured to use a Bluetooth protocol, such as a BLE protocol.

Item 16. Method according to any one of Items 14-15, wherein the first command comprises a first command identifier, and wherein to associate the first command with a first message comprises to associate the first command identifier with the first message.

Item 17. Method according to any of Items 14-16, wherein the first message is a pre-determined message associated with the first command.

Item 18. Method according to Item 17, wherein the first message is a pre-determined voice prompt or a pre-determined text prompt.

Item 19. Method according to any of Items 14-18, wherein the method comprises receiving, via the radio interface, a second radio signal in response to the first signal, and wherein transmitting the first output comprises transmitting the second radio signal via the hearing protection interface.

Item 20. Method according to any of Items 14-19, wherein outputting the first signal indicative of the first message via the radio interface comprises outputting the first signal via the radio interface to a radio unit of another user and/or to a server device.

Item 21. Method according to any of Items 14-20, the method comprising outputting the first message via the hearing protection interface before outputting the first signal via the radio interface.

Item 22. Method according to any of Items 14-21, the method comprising receiving a second command from the accessory device, where the second command is configured to confirm or cancel the first command.

Item 23. Method according to Item 22, wherein outputting the first signal indicative of the first message via the radio interface comprises outputting, in accordance with determining that the first command is indicative of a confirmation of the first command or the second command (cancellation) is not received within a time limit, the first signal indicative of the first message via the radio interface.

Item 24. Method according to Item 22, wherein the method comprises forgoing outputting the first signal indicative of the first message via the radio interface in accordance with determining that the second command is indicative of a cancellation of the first command or the second command (confirmation) is not received within a time limit.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

It may be appreciated that the figures comprise some components, circuitries, or operations which are illustrated with a solid line and some which are illustrated with a dashed line. Dashed lines are generally used to provide context and/or to depict further details or aspects not otherwise apparent from a 2D schematic. The depiction of solid or dashed lines, and the relationship to the various described embodiments, is to be understood in view of the corresponding description. Some connections between components are described but not depicted; the skilled person will recognize the interconnected or interoperability between components. It should be appreciated that these operations need not be performed in order presented. Furthermore, it should be appreciated that not all of the operations need to be performed. The example operations may be performed in any order and in any combination.

It is to be noted that the word "comprising" does not necessarily exclude the presence of other elements or steps than those listed.

It is to be noted that the words "a" or "an" preceding an element do not exclude the presence of a plurality of such elements.

It is to be noted that the term "indicative of" may be seen as "associated with", "related to", "descriptive of", "characterizing", and/or "defining". The terms "indicative of", "associated with", "related to", "descriptive of", "characterizing", and "defining" can be used interchangeably. The term "indicative of" can be seen as indicating a relation. For example, weight data indicative of weight may comprise one or more weight parameters.

It is to be noted that the word "based on" may be seen as "as a function of" and/or "derived from". The terms "based on" and "as a function of" can be used interchangeably. For example, a parameter determined "based on" a data set can be seen as a parameter determined "as a function of" the data set. In other words, the parameter may be an output of one or more functions with the data set as an input.

A list in the form of "A, B, or C, or any combination therefore" should be understood to mean "A", or "B", or "C", or "A and B", or "A and C", or "B and C", or "A and B and C".

A function may be characterizing a relation between an input and an output, such as mathematical relation, a database relation, a hardware relation, logical relation, and/or other suitable relations.

It should further be noted that any reference signs do not limit the scope of the claims, that the example embodiments may be implemented at least in part by means of both hardware and software, and that several "means", "units" or "devices" may be represented by the same item of hardware.

The various example methods, devices, nodes and systems described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program circuitries may include routines, programs, objects, components, data structures, etc. that perform specified tasks or implement specific abstract data types. Computer-executable instructions, associated data structures, and program circuitries represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed disclosure, and it will be made obvious to those skilled in the art that various changes and modifications may be made without departing from the scope of the claimed disclosure. The specification and drawings are, accordingly, to be regarded in an illustrative rather than restrictive sense. The claimed disclosure is intended to cover all alternatives, modifications, and equivalents.

### LIST OF REFERENCES

- 1: hearing protection system
- 2: hearing protection device
- 3A: first ear cup
- 3B: second ear cup
- 4: communication device
- 6: accessory device
- 8: radio unit
- 10: processing unit
- 12: memory
- 14: radio interface
- 16: hearing protection device interface
- 18: wireless interface
- 20: radio connector
- 22: cable
- 24: hearing protection device interface
- 26: cable
- 28: communication device connector
- 30: first input from hearing protection device
- 32: first output to hearing protection device
- 34: second input from radio unit
- 36: second output to radio unit
- 36A: first signal
- 40: wireless interface
- 42: processing unit
- 44: user interface
- 46: first button
- 48: second button
- 50: second radio signal
- 200: hearing protection system
- 100, 100A: method of operating a communication device for a hearing protection system
- S102: obtaining a first input from the hearing protection device
- S104: transmitting a first output to the hearing protection device
- S106: obtaining a second input from the radio unit
- S106A: transmitting the second radio signal via the hearing protection interface
- S108: transmitting a second output to the radio unit
- S110: receiving one or more commands comprising a first command from the accessory device
- S112: associating the first command with a first message
- S114: outputting a first signal indicative of the first message via the radio interface
- S114A: outputting the first signal via the radio interface to a radio unit of another user and/or to a server device
- S116: receiving a second radio signal in response to the first signal
- S118: outputting the first message via the hearing protection interface before outputting the first signal via the radio interface.
- S120: receiving a second command where the second command is configured to confirm or cancel the first command
- S122: determining whether the first command is ok or not

## Claims

1. A communication device (4) for a hearing protection system (1) comprising a hearing protection device (2), the communication device (4) comprising:
a processing unit (10);
a memory (12);
a radio interface (14); and
a hearing protection device interface (16),
wherein the processing unit is configured to obtain a first input from the hearing protection device and/or transmit a first output to the hearing protection device via the hearing protection device interface, and wherein the radio interface comprises a radio connector, where the radio connector comprises terminals for wired connection to a radio unit, the processing unit is configured to obtain a second input from the radio unit and/or transmit a second output to the radio unit via the radio interface, wherein the communication device comprises a wireless interface (18) for communicating with an accessory device, wherein the communication device is configured to receive one or more commands comprising a first command from the accessory device via the wireless interface, to associate the first command with a first message, and to output a first signal indicative of the first message via the radio interface.

2. Communication device according to claim 1, wherein the wireless interface is configured to use a Bluetooth protocol.

3. Communication device according to any one of claims 1-2, wherein the first command comprises a first command identifier, and wherein to associate the first command with a first message comprises to associate the first command identifier with the first message.

4. Communication device according to any of the previous claims, wherein the first message is a pre-determined message associated with the first command.

5. Communication device according to claim 4, wherein the first message is a pre-determined voice prompt or a pre-determined text prompt.

6. Communication device according to any of the previous claims, wherein the communication device is configured to receive, via the radio interface, a second radio signal in response to the first signal, and to transmit the first output comprises to transmit the second radio signal via the hearing protection interface.

7. Communication device according to any of the previous claims, wherein to output the first signal indicative of the first message via the radio interface comprises to output the first signal via the radio interface to a radio unit of another user and/or to a server device.

8. Communication device according to any of the previous claims, wherein the communication device is configured to output the first message via the hearing protection interface before outputting the first signal via the radio interface.

9. Communication device according to any of the previous claims, wherein the communication device is configured to receive a second command from the accessory device, where the second command is configured to confirm or cancel the first command.

10. An accessory device for a hearing protection system comprising a hearing protection device and a communication device, the accessory device comprising a processing unit, a wireless interface, and a user interface, wherein the processing unit is configured to detect a user input via the user interface, and transmit or output a first command to the communication device via the wireless interface in accordance with a detection of a first user input, wherein the first command is indicative of a first message.

11. Accessory device according to claim 10, wherein the user interface comprises one or more of a press button, a switch button, and a joystick.

12. Accessory device according to claim 11, wherein the accessory device is configured to be mounted on a weapon of a user of the communication device.

13. A hearing protection system comprising a communication device according to any of claims 1-9 and an accessory device according to any of claims 10-12.

14. A method of operating a communication device for a hearing protection system comprising a hearing protection device, the communication device comprising a processing unit, a memory, a radio interface comprising a radio connector, a hearing protection device interface, and a wireless interface, the method comprising:
obtaining, via the hearing protection device interface, a first input from the hearing protection device;
transmitting, via the hearing protection device interface, a first output to the hearing protection device;
obtaining, via the radio interface, a second input from the radio unit;
transmitting, via the radio interface, a second output to the radio unit;
receiving, via the wireless interface, one or more commands comprising a first command from the accessory device;
associating the first command with a first message; and
outputting a first signal indicative of the first message via the radio interface.
